# EUROPEAN PATENT SPECIFICATION

(11) **EP 0 536 853 B1**
(45) Date of publication and mention of the grant of the patent: **27.03.1996**
(21) Application number: 92203575.3
(22) Date of filing: 01.12.1987
(51) Int. Cl.: C07C 209/08, C07C 209/16

(54) **Process for the preparation of N1,N4-diethylspermine**
Verfahren zur Herstellung von N1,N4-Diethylspermin
Procédé pour la préparation de N1,N4-diéthylspermine

(30) Priority: 02.12.1986 US 936835
(43) Date of publication of application: 14.04.1993
(62) Divisional of application: 87310591.0
(73) Proprietor: THE UNIVERSITY OF FLORIDA, Gainesville, Florida 32611 (US)
(72) Inventor: Bergeron, Raymond J., Jr., Gainesville, Florida 32601 (US)
(74) Representative: Hedley, Nicholas James Matthew

(56) References cited:
- EP-A- 0 116 693
- JOURNAL OF MEDICINAL CHEMISTRY, vol. 16, no. 1, January 1973, pages 1-5, Washington, DC, US; M. ISRAEL et al.: "Synthesis and antitumor evaluation of the presumed cytotoxic metabolites of spermine and N, N'-bis(3- aminopropyl)nonane 1,9-diamine"
- JOURNAL OF BIOCHEMISTRY, vol. 100, no. 1, July 1986, pages 123-130, Tokyo, JP; M. TAKAHASHI et al.: "Thermophilic HB8 DNA ligase: effects of polyethylene glycols and polyamines on blunt-end ligation of DNA"
- CANCER RESEARCH, vol. 45, no. 5, May 1985, pages 2050-2057, Philadelphia, US; C.W. PORTER et al.: "Biological properties of N4- and N1,N8-spermidine derivatives in cultured L1201 leukemia cells"

## Description

This invention was made with U.S. Government support under Grant NCDDG-CA37606, awarded by the National Cancer Institute. The U.S. Government has certain rights in this invention.

### Field of the Invention

The present invention relates to anti-neoplastic pharmaceutical compositions.

### Prior Art

In recent years a great deal of attention has been focused on the polyamines, e.g., spermidine, norspermidine, homospermidine, 1,4-diaminobutane (putrescine), and spermine. These studies have been directed largely at the biological properties of the polyamines probably because of the role they play in proliferative processes. It was shown early on that the polyamine levels in dividing cells,e.g., cancer cells, are much higher than in resting cells. See Janne et al, A. Biochim. Biophys. Acta. 473, 241 (1978); Fillingame et al, Proc. Natl. Acad. Sci. U.S.A. 72:4042 (1975); Metcalf et al, J. Am. Chem. Soc. 100:2551 (1978); Flink et al, Nature (London) 253:62 (1975); and Pegg et al, Polyamine Metabolism and Function, Am. J. Cell. Physiol. 243:212-221 (1982).

Several lines of evidence indicate that polyamines, particularly spermidine, are required for cell proliferation: (i) they are found in greater amounts in growing than in non-growing tissues; (ii) prokaryotic and eukaryotic mutants deficient in polyamine biosynthesis are auxotrophic for polyamines; and (iii) inhibitors specific for polyamine biosynthesis also inhibit cell growth. Despite this evidence, the precise biological role of polyamines in cell proliferation is uncertain. It has been suggested that polyamines, by virtue of their charged nature under physiological conditions and their conformational flexibility, might serve to stabilize macromolecules such as nucleic acids by anion neutralization. See Dkystra et al, Science, 149:40 (1965); Russell et al, Polyamines as Biochemical Markers of Normal and Malignant Growth (Raven, New York, 1978); Hirschfield et al, J. Bacteriol., 101:725 (1970); Morris et al, ibid, p. 731; Whitney et al, ibid, 134:214 (1978); Hafner et al, J. Biol. Chem., 254:12419 (1979); Cohn et al, J. Bacteriol. 134:208 (1978); Pohjatipelto et al, Nature (London), 293:475 (1981); Mamont et al, Biochem. Biophys. Res. Commun. 81:58 (1978); Bloomfield et al, Polyamines in Biology and Medicine (D.R. Morris and L.J.. Morton, Eds. - Dekker, New York, 1981) pp. 183-205; Gosule et al, Nature, 259:333 (1976); Gabbay et al, Ann. N.Y. Acad. Sci., 171:810 (1970); Suwalsky et al, J. Mol. Biol., 42:363 (1969) and Liquori et al, J. Mol. Biol., 24:113 (1968).

However, regardless of the reason for increased polyamine levels the phenomenon can be and has been exploited in chemotherapy. See Sjoerdsma et al, Butterworths Int. Med. Rev.: Clin. Pharmacol. Ther. 35:287 (1984); Israel et al, J. Med. Chem., 16:1 (1973); Morris et al, Polyamines in Biology and Medicine; Dekker, New York, p. 223 (1981) and Wang et al, Biochem. Biophys. Res. Commun., 94:85 (1980).

J. Biochemisty, 100 (1), pp. 123-130 (1986) discloses N¹, N⁴⁻diethylspermine and its use in a DNA ligation process.

Reference is made to copending Application No. 87310591.1 (Publication No. 0 270 349) which provides novel anti-neoplastic pharmaceutical compositions.

### SUMMARY OF THE INVENTION

The present invention provides a process of making N¹,N⁴-diethylspermine having the formula:

**CH₃CH₂-N¹H-(CH₂)₃-N²H-(CH₂)₄-N³H-(CH₂)₃-N⁴H-CH₂CH₃.**

and pharmaceutically acceptable acid addition salts thereof, as defined in the accompanying claims.

### DETAILED DESCRIPTION OF THE INVENTION

It is disclosed in EP-A-0,270,349 that N¹^{,}N⁴-diethylspermine and pharmaceutically acceptable acid addition salts thereof are active against malignant cells in vitro and in vivo in human and non-human animals.

The compound is preferably synthesized by first forming a sulfonamide of the polyamine at all of the amino nitrogens (1) to activate the primary amines for mono ethylation, and (2) to protect any secondary nitrogens from ethylation. Suitable sulfonating agents include alkyl, aryl and arylalkyl sulfonating agents of the general structure RSO₂X wherein R is alkyl, aryl or arylalkyl and X is a leaving group, e.g., Cl⁻, Br⁻, etc. The sulfonation is accomplished by reacting the polyamine with 1.0 equivalent of sulfonating agent per nitrogen in the presence of a base, e.g. tertiary amine or a hydroxide. The reaction is best accomplished using aqueous sodium hydroxide as the base and p-toluenesulfonyl chloride (TsCl) as the sulfonating agent in a biphasic solvent system consisting of an organic solvent, e.g. methylene chloride, and water. The sulfonating agent is added in methylene chloride to an aqueous solution of the amine and sodium hydroxide and the reaction proceeds according to the following equation, using spermine as the base compound: 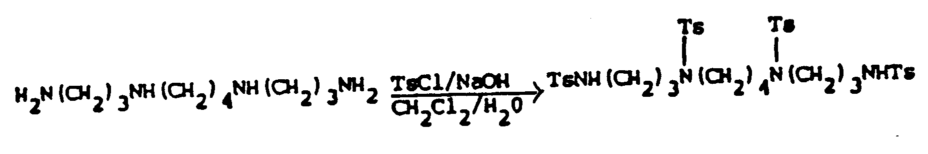
**Wherein: Ts = p-toluenesulfonyl.**

After purification the sulfonamide is next ethylated. The ethylation involves formation of N-anions on the primary amino sulfonamides with a base such as NaH followed by reaction of the N-anion with an ethylating agent C₂H₅X wherein X is a leaving group such as I⁻, CI⁻, Br⁻, p-CH₃C₆H₄SO₃⁻, CH₃SO₃⁻.

The ethylation can be carried out in a variety of dipolar aprotic solvents, preferably, N, N-dimethylformamide (DMF). The reaction proceeds according to the following equation:

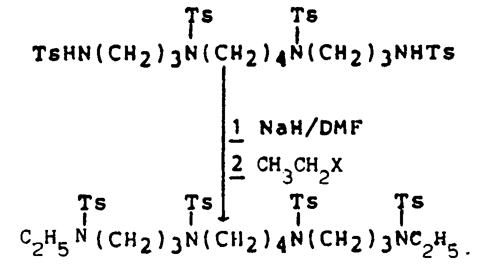

After ethylation of the sulfonamide, the sulfonyl protecting groups are next removed under reducing conditions. Although a variety of standard reducing conditions can be utilized (LiAlH₄, Li/NH₃, catalytic reduction), Na and NH₃ function optimally. The reduction proceeds according to the following equation:

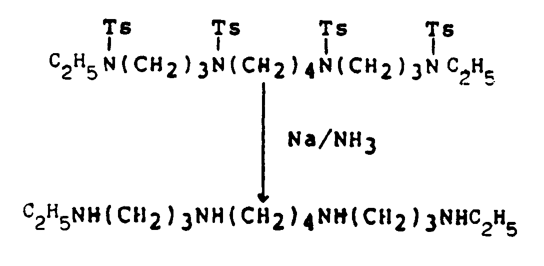

The compound is isolated as the free amine and then may be converted to and utilized as the corresponding hydrochloride salt by treatment with concentrated HCl. However, it may also be used as salts with any pharmaceutically acceptable acid, e.g., HBr, CH₃CO₂H, CH₃SO₃H, etc.

The invention is illustrated by the following non-limiting example.

### EXAMPLE

### Preparation of N¹,N⁴-diethylspermine

N¹,N²,N³,N⁴-Tetra-p-tosylspermine. To spermine tetrahydrochloride (4.53 g, 13.0 mmol) and 10% aqueous NaOH (200 mL, 132 mmol) at 0° is added dropwise p-toluenesulfonyl chloride (9.98 g, 52.3 mmol) in CH₂Cl₂ with rapid stirring. After 1 hr the mixture is allowed to warm to room temperature and to stir for 2 days. The organic phase is separated and washed with 0.5 N HCl, H₂O, and brine, dried over Na₂SO₄ and purified on silica gel (450 g, 3% MeOH/CHCl₃) to give 9.69 g, 91% yield of tetratosylspermine.
NMR (CDCl₃) δ 7.2-7.9 (m, 16H), 5.34 (t, 2H, J=7), 2.9-3.3 (m, 12H), 2.43 (s, 12H), 1.5-2.0 (m, 8H).
N¹,N⁴-Diethyl-N¹,N²,N³,N⁴-Tetra-p-tosylspermine. To the tetratosylspermine prepared above (1.75 g, 2.14 mmol) in dry DMF, (12 mL) was cautiously added 80% sodium hydride (0.25 g, 8.33 mmol) and then ethyl iodide (1.0 mL, 12.5 mmol). After heating under nitrogen (10 h, 55°), the mixture was quenched with ice water and extracted with chloroform (3 x). The organic phase was washed with 5% Na₂SO₃, 5% NaOH, 1N HCl, and water, then dried with Na₂SO₄. Removal of DMF by flash distillation and purification of the crude product on silica gel (4%EtOH/CHCl₃) produced 1.63 g (87%) of the desired product. NMR (CDCl₃) δ 7.2-7.8 (m, 16H), 3.0-3.3 (m, 16H), 2.43 (s, 12H), 1.5-2.1 (m, 8H), 1.08 (t, 6H, J=7). Anal. Calcd. for C₄₂H₅₈N₄O₈S₄; C, 57.64; H, 6.68; N, 6.40. Found: C, 57.69; H, 6.74; N, 6.20.
N¹,N⁴-diethylspermine (DES). Into a solution of the N¹,N⁴-diethyl-N¹,N²,N³,N⁴-tetratosylspermine prepared above (2.78 g, 3.18 mmoles) in dry, distilled THF (200 mL) at -78°C. was condensed 300 mL NH₃, using a dry ice condenser. Sodium spheres (3.0 g, 0.13 mol) were then added in small portions and the reaction mixture was stirred at -78°C. for 4 hr. The reaction mixture was allowed to warm to room temperature overnight and the NH₃ boiled off. Diethyl ether was added to the mixture. Ethanol was then cautiously added, then H₂O was added to finally quench the reaction. The solvents were evaporated and the product extracted with diethyl ether and then chloroform. The extracts were dried over Na₂SO₄, filtered and the extracts concentrated. The resultant liquid was distilled in a Kugelrohr apparatus (150°C., 0.1 mm). Concentrated hydrochloric acid was added to an ether/ethanol (1:1) solution of the distillate to form the hydrochloride salt which was recrystallized from hot aqueous ethanol to give 790 mg (63%) DES. NMR (D₂O) δ 1.4 (t, 6H); 1.9 (m, 4H); 2.25 (m, 4H); 3.25 (m, 16H); 4.80 (s, HOD, reference).

Results demonstrating the therapeutic effect of N¹,N⁴-diethylspermine are set out in EP-A-0 270 349.

## Claims

1. A process of making N¹,N⁴diethylspermine having the general formula:
**CH₃CH₂-N¹H-(CH₂)₃-N²H-(CH₂)₄-N³H-(CH₂)₃-N⁴H-CH₂CH₃.**
and pharmaceutically acceptable salts thereof by reacting spermine:
N¹H₂-(CH₂)₃-N²H-(CH₂)₄-N³H-(CH₂)₃-N⁴H₂
with a sulfonating agent of the general formula RSO₂X, where X stands for a leaving group and RSO₂ stands for a sulfonyl protecting group, e.g. an alkyl-, aralkyl- or aryl- sulfonyl protecting group, in the presence of a base, reacting the compound thus produced: 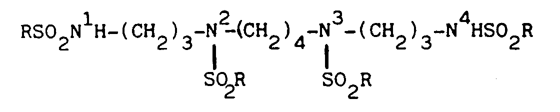 with a strong base, e.g. NaH, and an ethylating agent in an aprotic dipolar solvent to produce a compound of the general formula: 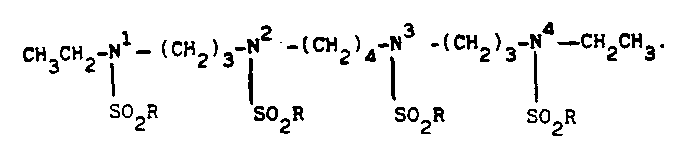 and removing the protecting groups under reducing conditions to provide N¹,N⁴ - diethylspermine, which optionally is converted into a pharmaceutically acceptable acid addition salt.

2. A process as claimed in claim 1, wherein the base used in the sulphonation of spermine comprises a tertiary amine or a hydroxide, e.g. sodium hydroxide.

3. A process as claimed in claim 1 or claim 2, wherein the sulphonation of spermine is carried out in a biphasic reaction medium having an organic and an aqueous phase, the sulphonating agent being added in the organic phase and the spermine and the base being added in the aqueous phase.

4. A process as claimed in any one of claims 1 to 3, wherein, in the sulphonation of spermine, the base is aqueous sodium hydroxide and the sulphonating agent is tosyl chloride.

5. A process as claimed in any one of claims 1 to 4, wherein the protecting groups are removed using sodium and ammonia.

## Patentansprüche

1. Verfahren zur Herstellung von N¹,N⁴-Diethylspermin mit der allgemeinen Formel:
CH₃CH₂-N¹H-(CH₂)₃-N²H-(CH₂)₄-N³H-(CH₂)₃-N⁴H-CH₂CH₃
und pharmazeutisch annehmbare Salze davon durch Umsetzen von Spermin:
N¹H₂-(CH₂)₃-N²H-(CH₂)₄-N³H-(CH₂)₃-N⁴H₂
mit einem Sulfonierungsmittel der allgemeinen Formel RSO₂X, worin S für eine Abgangsgruppe und RSO₂ für eine Sulfonyl-Schutzgruppe steht, z.B. eine Alkyl-, Aralkyl- oder Aryl-Sulfonyl-Schutzgruppe, in Gegenwart einer Base, Umsetzen der derart hergestellten Verbindung: 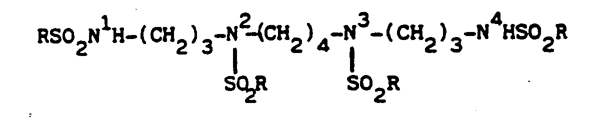 mit einer starken Base, z.B. NaH, und einem Ethylierungmittel in einem aprotischen, dipolaren Lösungsmittel, um eine Verbindung der allgemeinen Formel: 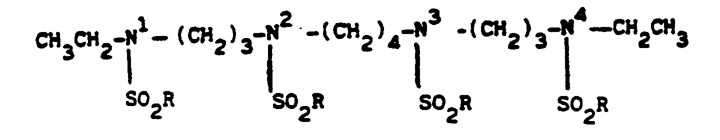 herzustellen, und Entfernen der Schutzgruppen unter reduzierenden Bedingungen, um N¹,N⁴-Diethylspermin bereitzustellen, welches gegebenenfalls zu einem pharmazeutisch annehmbaren Säureadditionssalz umgewandelt wird.

2. Verfahren nach Anspruch 1, worin die bei der Sulfonierung von Spermin verwendete Base ein tertiäres Amin oder ein Hydroxid, z.B. Natriumhydroxid, umfaßt.

3. Verfahren nach Anspruch 1 oder Anspruch 2, wobei die Sulfonierung von Spermin in einem biphasischen Reaktionsmedium mit einer organischen und einer wäßrigen Phase durchgeführt wird, wobei das Sulfonierungsmittel der organischen Phase zugesetzt wird und das Spermin und die Base der wäßrigen Phase hinzugesetzt werden.

4. Verfahren nach einem der Ansprüche 1 bis 3, wobei bei der Sulfonierung von Spermin die Base wäßriges Natriumhydroxid und das Sulfonierungsmittel Tosylchlorid ist.

5. Verfahren nach einem der Ansprüche 1 bis 4, wobei die Schutzgruppen unter Verwendung von Natrium und Ammoniak entfernt werden.

## Revendications

1. Procédé de fabrication de la N¹,N⁴-diéthylspermine de formule générale :
CH₃CH₂-N¹H-(CH₂)₃-N²H-(CH₂)₄-N³H-(CH₂)₃-N⁴H-CH₂CH₃.
et des sels pharmaceutiquement acceptables de celle-ci, par réaction de la spermine :
N¹H₂-(CH₂)₃-N²H-(CH₂)₄-N³H-(CH₂)₃-N⁴H₂
avec un agent de sulfonation de formule générale RSO₂X, où X représente un groupe labile et RSO₂ représente un groupe protecteur de la fonction sulfonyle, par exemple, un groupe protecteur alkyl-, aralkyl- ou aryl-sulfonyle, en présence d'une base, réaction du composé ainsi produit : 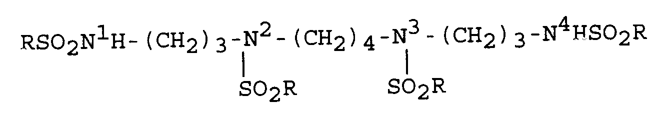 avec une base forte, par exemple NaH, et un agent d'éthylation dans un solvant dipolaire aprotique pour produire un composé de formule générale : 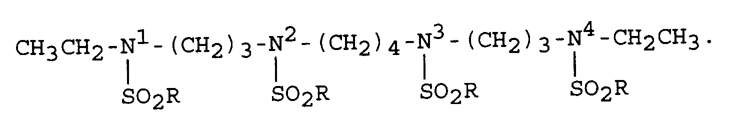 et enlèvement des groupes de protection dans des conditions réductrices pour donner la N¹,N⁴-diéthylspermine qui est éventuellement transformée en un sel d'addition acide pharmaceutiquement acceptable.

2. Procédé selon la revendication 1, dans lequel la base utilisée dans la sulfonation de la spermine est formée d'une amine tertiaire ou d'un hydroxyde, par exemple l'hydroxyde de sodium.

3. Procédé selon la revendication 1 ou 2, dans lequel la sulfonation de la spermine est mise en oeuvre dans un milieu réactionnel à deux phases, ayant une phase organique et une phase aqueuse, l'agent de sulfonation étant ajouté dans la phase organique et la spermine et la base étant ajoutées dans la phase aqueuse.

4. Procédé selon l'une quelconque des revendications 1 à 3, dans lequel, pour la sulfonation de la spermine, la base est une solution aqueuse d'hydroxyde de sodium et l'agent de sulfonation est le chlorure de tosyle.

5. Procédé selon l'une quelconque des revendications 1 à 4, dans lequel les groupes protecteurs sont éliminés en utilisant du sodium et de l'ammoniaque.
